(19) **Europäisches Patentamt / European Patent Office / Office européen des brevets**

(11) **EP 1 749 541 B1**

(12) **EUROPEAN PATENT SPECIFICATION**

(45) Date of publication and mention
of the grant of the patent:
**24.09.2014 Bulletin 2014/39**

(21) Application number: **05743791.5**

(22) Date of filing: **27.05.2005**

(51) Int Cl.:
*A61K 47/36* (2006.01)     *A61K 9/08* (2006.01)
*A61K 47/38* (2006.01)     *A61P 27/02* (2006.01)

(86) International application number:
**PCT/JP2005/009727**

(87) International publication number:
**WO 2005/115475 (08.12.2005 Gazette 2005/49)**

(54) **Eyedrops**

Augentropfen

Gouttes ophtalmologiques

(84) Designated Contracting States:
**AT BE BG CH CY CZ DE DK EE ES FI FR GB GR
HU IE IS IT LI LT LU MC NL PL PT RO SE SI SK TR**

(30) Priority: **27.05.2004  JP 2004157574
27.08.2004  JP 2004248586**

(43) Date of publication of application:
**07.02.2007  Bulletin 2007/06**

(73) Proprietor: **Santen Pharmaceutical Co., Ltd
Osaka-shi, Osaka 533-8651 (JP)**

(72) Inventors:
 • **SAKAMOTO, Kayoko,**
   Santen Pharmaceutical Co., Ltd.
   Osaka-shi,
   Osaka 5338651 (JP)
 • **KIMURA, Akio,**
   c/o Santen Pharmaceutical Co., Ltd.
   Osaka-shi,
   Osaka 5338651 (JP)
 • **HIRAI, Shin-ichiro,**
   Santen Pharmaceutical Co., Ltd
   Ikoma-shi,
   Nara 6300101 (JP)
 • **NAKAMURA, M.,**
   Santen Pharmaceutical Co., Ltd.
   Ikoma-shi,
   Nara 6300101 (JP)

(74) Representative: **Müller-Boré & Partner
Patentanwälte PartG mbB
Friedenheimer Brücke 21
80639 München (DE)**

(56) References cited:
    WO-A2-02/58668     WO-A2-03/015809
    JP-A- 05 508 840

 • **LUDWIG A. ET AL.: 'The evaluation of viscous
   ophtalmic vehicles by slit lamp fluorophotometry
   in humans.' INT.PHARM. vol. 54, 1989, pages 95
   - 102, XP002997050**
 • **AKIRA NAKAJIMA ET AL.: 'Kakushu Kakumaku
   Naihi Hogozai ni tsuite.' GANKI vol. 35, 1984,
   pages 1862 - 1868, XP002997051**

EP 1 749 541 B1

**Description**

Technical Field

The present invention relates to a viscous agent for ophthalmic use comprising a combination of hydroxyethylcellulose and dextran. Further, the present invention relates to an eyedrop containing a viscous agent for ophthalmic use comprising the combination of hydroxyethylcellulose and dextran, characterized in that it is thickened upon contact with mucin in the precorneal tear film at instillation. Further, the present invention relates to a precorneal tear film stabilizer and an enhancer for retention of eyedrop on the ocular surface, comprising the combination of hydroxyethylcellulose and dextran.

Background Art

It is known that an eyedrop is retained on the ocular surface for a long time and a desired pharmacological efficacy, refreshing feel or the like is sustained. When it has high viscosity, however, as the viscosity of an eyedrop is higher, it is more difficult to instill the eyedrop and feel in instillation is deteriorated.

The ocular surface is covered with the precorneal tear film composed of three layers, i.e., an oily layer, an aqueous layer and a mucin layer. The mucin layer located at the innermost layer of the precorneal tear film plays a role in allowing the precorneal tear film to adhere to the ocular surface so as not to flow tears off. When the precorneal tear film becomes unstable due to disorder of the mucin layer, a dry area called "dry spot" appears within a short period of time just before blinking, therefore, dry eye symptoms accompanying dry feel or uncomfortable feel in the ocular area may be caused in some cases.

On the other hand, hydroxyethylcellulose is a cellulose derivative having a hydroxyethoxyl group, and is generally used as a viscous agent or a dispersant. Further, dextran is a polysaccharide obtained by fermentation of sucrose, and is generally used as a viscous agent or a stabilizer for an eyedrop.

JP-A-2002-97129 discloses an invention which relates to an eyedrop comprising a refreshing component and a viscous agent, and examples of the viscous agent include ethylcellulose, hydroxyethylcellulose, dextran and the like. US Patent No. 6572849 discloses an invention which relates to a composition for ophthalmic use with a pH of 1.5 to 3.5 having a self-preserving ability, and as additives thereof, polyethylene glycol, hydroxypropylmethylcellulose, hydroxyethylcellulose, dextran and the like are described therein.

However, neither one of the above-mentioned documents is directed to the one in which hydroxyethylcellulose and dextran are formulated in combination.

Disclosure of the invention

Problems that the Invention is to Solve

With regard to the viscosity of an eyedrop, when an eyedrop with low viscosity is instilled, it immediately flows out of the ocular surface, therefore, a desired pharmacological efficacy or refreshing feel cannot be sustained. On the other hand, when the viscosity of an eyedrop is high, it is retained on the ocular surface for a long time. However, there is a tendency that the higher the viscosity of an eyedrop is, the more difficult the instillation is and feel in instillation is deteriorated. Accordingly, there has been a demand for development of an eyedrop which has relatively low viscosity before instillation and is thickened on the ocular surface in instillation thereby to sustain a desired pharmacological efficacy or refreshing feel. There has also been a demand for development of an eyedrop which can stabilize the precorneal tear film thereby to relieve dry feel and uncomfortable feel in the ocular area.

Means for Solving the Problems

The present inventors made intensive studies by focusing attention on the fact that when an eyedrop is instilled, it comes into contact with the precorneal tear film on the ocular surface, in particular mucin in the precorneal tear film. As a result, it was found that an eyedrop containing a viscous agent for ophthalmic use comprising a combination of hydroxyethylcellulose and dextran exhibits a specific and synergistic thickening effect in the coexistence of mucin, and thus the present invention has been achieved. Further, the eyedrop containing a viscous agent for ophthalmic use of the present invention has an excellent effect of stabilizing the precorneal tear film, which was found from the results of a test on changes of corneal surface regularity index, which will be described later, therefore it is useful as a therapeutic agent for dry eyes or artificial tears. Further, the viscous agent for ophthalmic use of the present invention can also be used as a vehicle for an eyedrop that provides comfortable feel in instillation or an eye-friendly eyewash.

The eyedrop containing a viscous agent for ophthalmic use of the present invention exhibits a specific and synergistic thickening effect in the coexistence of mucin, which is presumed to be based on the event that the binding of the viscous agent for ophthalmic use comprising a combination of hydroxyethylcellulose and dextran to mucin on the ocular surface is enhanced. When the viscous agent for ophthalmic use of the present invention is used as a vehicle for an eyedrop, the eyedrop has an excellent action of enhancing retention of eyedrop on the ocular surface, which was found from the results of a test for evaluation of ocular surface retention of eyedrop, which will be described later. Accordingly, it can be also applied to a drug delivery system which enhances the retention of drugs in anterior segment of the eye and intraocular penetration of drugs.

The present invention relates to:

An eyedrop containing a viscous agent for ophthalmic use comprising a combination of hydroxyethylcellulose and dextran,
wherein the pH thereof is in the range of 4 to 8, and
wherein the concentration of hydroxyethylcellulose is in the range of 0.001 to 10% (w/v) and the concentration of dextran is in the range of 0.001 to 10% (w/v);
in that the eyedrop is thickened upon contact with mucin in a precorneal tear film at instillation;

The eyedrop comprising a combination of hydroxyethylcellulose and dextran of the present invention exhibits a specific and synergistic thickening effect in the presence of mucin, therefore, the eyedrop is thickened on the ocular surface upon contact with mucin in the precorneal tear film at instillation, whereby a desired pharmacological efficacy or refreshing feel can be sustained. Further, since the eyedrop of the present invention stabilizes the precorneal tear film, it is useful for relieving dry feel or uncomfortable feel in the ocular area, which leads to improvement of dry eye symptoms. As will be described in detail later in the section of "test for measurement of viscosity in the coexistence of mucin", while a viscous agent comprising a combination of hydroxyethylcellulose and dextran exhibits a specific and synergistic thickening action in the presence of mucin, a viscous agent comprising a combination of hydroxypropylmethylcellulose with dextran, which is another cellulosic viscous agent, does not exhibit a synergistic thickening action. Accordingly, it is found that the synergistic thickening action is based on a specific property of hydroxyethylcellulose.

In the viscous agent, the precorneal tear film stabilizer and the enhancer for retention of eyedrop on the ocular surface comprising a combination of hydroxyethylcellulose and dextran according to the present invention, the weight ratio of hydroxyethylcellulose to dextran is preferably 0.001 to 10 of hydroxyethylcellulose to 0.001 to 10 of dextran, more preferably 0.01 to 5 of hydroxyethylcellulose to 0.005 to 3 of dextran.

The hydroxyethylcellulose to be used in the present invention is a cellulosic polymer and is available in various viscosities, and any of them can be used. The concentration of hydroxyethylcellulose in the eyedrop is not particularly limited. However, from the viewpoint of the properties or effects of the eyedrop, it is in the range of 0.001 to 10% (w/v), more preferably in the range of 0.01 to 5% (w/v).

The dextran to be used in the present invention is a polysaccharide obtained by fermentation of sucrose and is available in various average molecular weights, and any of them can be used. Preferred examples include dextran 40 (average molecular weight of about 40,000) and dextran 70 (average molecular weight of about 70,000). The concentration of dextran in the eyedrop is not particularly limited. However, from the viewpoint of the properties or effects of the eyedrop, it is in the range of 0.001 to 10% (w/v), more preferably in the range of 0.005 to 3% (w/v).

The eyedrop containing comprising a combination of hydroxyethylcellulose and dextran according to the present invention can be prepared using a widely used technique. Preferred preparations are eyedrops.

In the eyedrop of the present invention, a drug can be formulated. Examples of the drug include an antibacterial agent, an antiinflammatory, an antihistamine, an antiglaucoma agent, an antiallergic agent, an immunosuppressive agent, an antimetabolite and the like.

In the eyedrop of the present invention, a tonicity agent, a buffer, a pH adjustor, a solubilizer, a stabilizer, a preservative or the like can be added as needed.

Examples of the tonicity agent include glycerol, propylene glycol, sodium chloride, potassium chloride, sorbitol, mannitol and the like.

Examples of the buffer include phosphoric acid, citric acid, acetic acid, ε-aminocaproic acid, boric acid, borax, trometamol and the like.

Examples of the pH adjustor include hydrochloric acid, citric acid, phosphoric acid, acetic acid, sodium hydroxide, potassium hydroxide, boric acid, borax, sodium carbonate, sodium hydrogencarbonate and the like.

Examples of the solubilizer to be added in the case, for example, where a drug or another additive is hardly soluble in water include polysorbate 80, polyoxyethylene hydrogenated castor oil 60, macrogol 4000 and the like.

Examples of the stabilizer include edetic acid, sodium edetate and the like.

Examples of the preservative include sorbic acid, potassium sorbate, benzalkonium chloride, benzethonium chloride, methyl p-oxybenzoate, propyl p-oxybenzoate, chlorobutanol and the like. These preservatives can also be used in combination.

The pH of the eyedrop of the present invention is preferably set within the range of 4.0 to 8.0. In addition, the osmotic pressure rate is preferably set at around 1.0.

The instillation frequency of the eyedrop of the present invention can properly be selected depending on the symptoms, age, dosage form or the like, however, it may be instilled once to about six times daily.

Advantageous effects of the Invention

As will be described in detail later in the section of "test for measurement of viscosity in the coexistence of mucin" , the eyedrop comprising a combination of hydroxyethylcellulose and dextran of the present invention exhibits a specific and synergistic thickening action in the coexistence of mucin. Therefore, the eyedrop of the present invention is thickened on the ocular surface upon contact with mucin in the precorneal tear film at instillation, whereby a desired pharmacological efficacy, refreshing feel or the like can be sustained. Further, as is apparent from the results of a test for evaluation of ocular surface retention of eyedrop and a test on changes of corneal surface regularity index, the eyedrop containing the viscous agent for ophthalmic use of the present invention is excellent in the retention of drugs in anterior segment of the eye and has an effect of stabilizing the precorneal tear film thereby to relieve dry feel in the ocular area. Therefore, it is useful for relieving dry feel or uncomfortable feel in the ocular area as an enhancer for retention of eyedrop on the ocular surface, a precorneal tear film stabilizer, a therapeutic agent for dry eyes or artificial tears. Further, it is also useful as a vehicle for an eyedrop that provides comfortable feel in instillation or an eye-friendly eyewash.

Best Mode for Carrying Out the Invention

Hereinafter, the present invention will be described in detail by referring to Examples, however, these examples are for understanding the invention well, and are not meant to limit the scope of the invention.

[1] Test for measurement of viscosity in the coexistence of mucin

This test is to evaluate a synergistic thickening action in the case where test solutions are brought into contact with a mucin solution respectively by measuring the viscosity in the coexistence of each test solution and mucin in accordance with the test method described in Pharmaceutical Research, 491-495, Vol. 7, No. 5, 1990.

(Experimental method)

As shown in Table 1, physiological saline was prepared and test solutions 1 to 6 were prepared by dissolving each viscous agent in physiological saline. Further, mucin (manufactured by SIGMA) was dissolved in 0.1 M phosphate buffer to give a pH equal to that of tears (neutral pH), whereby 4% mucin solution (pH 7.0) was prepared.

[Table 1]

|  | Viscous agent (%) |
| --- | --- |
| Test solution 1 | physiological saline (control) |
| Test solution 2 | hydroxyethylcellulose (0.2) |
| Test solution 3 | hydroxypropylmethylcellulose (0.25) |
| Test solution 4 | dextran (0.1) |
| Test solution 5 | hydroxypropylmethylcellulose (0.25) + dextran (0.1) |
| Test solution 6 | hydroxyethylcellulose (0.2) + dextran (0.1) |

Then, using a rotary viscometer (Rheostress RS100, manufactured by HAAKE), the viscosities ($\eta$1, $\eta$2 and $\eta$3) at a shear rate of 100 $S^{-1}$ when the shear rate was increased from 0.1 to 150 $S^{-1}$ in 2.5 minutes were measured for respective solutions of a mixed solution of each test solution (2 mL) and 4% mucin solution (6 mL), a mixed solution of each test solution (2 mL) and 0.1 M phosphate buffer (6 mL) and a mixed solution of water (2 mL) and 4% mucin solution (6 mL).

$\eta$1: viscosity of a mixed solution of each test solution (2 mL) and 4% mucin solution (6 mL)
$\eta$2: viscosity of a mixed solution of each test solution (2 mL) and 0.1 M phosphate buffer (6 mL)

$\eta 3$: viscosity of a mixed solution of water (2 mL) and 4% mucin solution (6 mL)

(Results)

In accordance with the following equation, the viscosity increase rate of each mixed solution was calculated. These results are shown in Table 2.

$$\texttt{Viscosity increase rate = (}\eta\texttt{1 - }\eta\texttt{2 - }\eta\texttt{3) / }\eta\texttt{2 } \times \texttt{ 100}$$

Incidentally, in the case where $\eta 1$ is substantially the same as $\eta 2$ and $\eta 3$, that is, there is almost no change in viscosity, the viscosity increase rate calculated from the above equation is around -100%. Further, in the case where $\eta 1$ obviously increases compared with $\eta 2$ and $\eta 3$, that is, $\eta 1$ is synergistically thickened, the viscosity increase rate calculated from the above equation becomes a positive value.

[Table 2]

| Test solution | Viscosity increase rate (%) |
| --- | --- |
| Test solution 1 | -105 |
| Test solution 2 | -31 |
| Test solution 3 | -21 |
| Test solution 4 | -93 |
| Test solution 5 | -47 |
| Test solution 6 | +22 |

(Discussion)

In the case where a viscous agent comprising a combination of hydroxyethylcellulose and dextran was allowed to coexist with mucin, a specific and synergistic thickening action was exhibited (test solution 6). However, when physiological saline, hydroxyethylcellulose, hydroxypropylmethylcellulose or dextran was allowed to coexist with mucin respectively, a synergistic thickening action was not exhibited (test solutions 1 to 4). Further, when a mixture of hydroxypropylmethylcellulose and dextran was allowed to coexist with mucin, a synergistic thickening action was not exhibited (test solution 5).

[2] Test on changes of corneal surface regularity index

In this test, corneal surface irregularities (irregularities of the precorneal tear film) after instilling each test solution were measured with a corneal shape measurement device for the purpose of evaluating a precorneal tear film stabilizing effect of each test solution.

(Experimental method)

A 20 $\mu$L portion of the test solution 1 or the test solution 6 was instilled to male Japanese white rabbits under systemic anesthesia, followed by measuring of the corneal surface shapes at 0 (immediately after instillation) and 10 minutes after instillation with the eyelids forcibly retracted, with a corneal shape measurement device (TMS-2N, manufactured by Tomey Corporation).

(Results)

Based on the measured surface regularity index (surface regularity index is increased with an increase in irregularity of the shape of the precorneal tear film on the corneal surface), surface regularity index changes (a surface regularity index change means a value obtained by subtracting the surface regularity index immediately after instillation from the surface regularity index 10 minutes after instillation) were calculated. The results are shown in Table 3. Note that the surface regularity index change of each of the test solutions is an average value of the three or five values.

[Table 3]

| Test solution | Change of surface regularity index after 10 minutes |
|---|---|
| Test solution 1 | 0.81 |
| Test solution 6 | 0.37 |

(Discussion)

As is apparent from Table 3, the test solution 6 containing a viscous agent comprising a combination of hydroxyethylcellulose and dextran exhibited a significant effect of stabilizing the precorneal tear film compared with physiological saline (test solution 1).

[3] Test for evaluation of retention of eyedrop on ocular surface

This test is to evaluate an action of ocular surface retention of each test eyedrop by measuring the fluorescence intensity in the precorneal tear film with a fluorophotometer for the anterior segment of the eye after the test eyedrop was instilled.

(Experimental method)

A 20 $\mu$L portion of the test solution 1 or the test solution 6 containing 0.002% sodium fluorescein respectively was instilled to male Japanese white rabbits under systemic anesthesia, followed by measuring of the fluorescence intensity in the precorneal tear film at every 30 seconds from 0 minute (immediately after instillation) to 5 minutes after instillation, at every 1 minute from 5 minutes to 10 minutes after instillation and at every 5 minutes from 10 minutes to 30 minutes after instillation with the eyelids forcibly retracted, with a fluorophotometer for the anterior segment of the eye (FL-500, Kowa).

(Results)

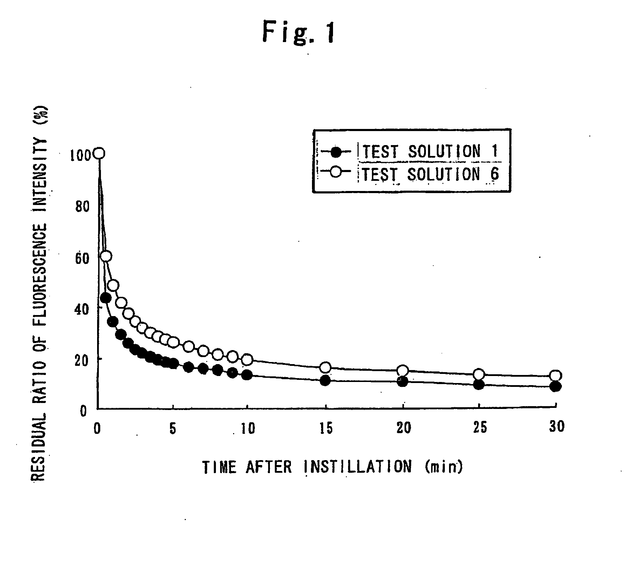

Based on the measured fluorescence intensity in the precorneal tear film, the average value of residual ratio of fluorescence intensity (which is a ratio of fluorescence intensity in each measurement time, when the fluorescence intensity immediately after instillation is assumed 100%) was calculated (an average of four cases each). Further, a noncompartmental analysis was carried out using the calculated average value, and $\lambda z$ (which indicates an elimination rate, wherein the smaller the value is, the longer the retention is) in the period from immediately after instillation to 2 minutes after instillation and AUC (which indicates an area under the fluorescence intensity vs. time curve, wherein the larger the value is, the longer the retention is) in the period from immediately after instillation to 30 minutes after instillation were calculated. The results of the measurement of fluorescence intensity and the results of the analysis are shown in Fig. 1 and Table 4, respectively.

[Table 4]

| Eyedrop | $\lambda z_{0-2}$ | $AUC_{0-30}$ |
|---|---|---|
| Test solution 1 | 0.6194 | 431.3 |
| Test solution 6 | 0.4639 | 611.7 |

(Discussion)

As is apparent from Fig. 1 and Table 4, the test solution 6 containing a viscous agent comprising a combination of hydroxyethylcellulose and dextran exhibited high ocular surface retention compared with physiological saline (test solution 1).

[Formulation examples]

Representative formulation examples obtained by formulating a viscous agent for ophthalmic use comprising a combination of hydroxyethylcellulose and dextran are shown below.

Formulation example 1 (eyedrop)

In 100 ml,

| | |
|---|---|
| hydroxyethylcellulose | 500 mg |
| dextran | 300 mg |
| sodium dihydrogenphosphate dihydrate | q.s. |
| 1 N sodium hydroxide | q.s. |
| hydrochloric acid | q.s. |
| sterile purified water | q.s. |

An eyedrop containing 100 mg, 1 g, 3 g or 5 g of hydroxyethylcellulose in 100 ml can be prepared in the same manner as the above formulation example.

Formulation example 2 (eyedrop)

In 100 ml,

| | |
|---|---|
| hydroxyethylcellulose | 300 mg |
| dextran | 300 mg |
| sodium dihydrogenphosphate dihydrate | q.s. |
| 1 N sodium hydroxide | q.s. |
| hydrochloric acid | q.s. |
| sterile purified water | q.s. |

An eyedrop containing 50 mg, 100 mg, 500 mg or 1 g of dextran in 100 ml can be prepared in the same manner as the above formulation example.

Brief Description of the Drawing

[Fig. 1] Fig. 1 is a graph showing a relationship between residual ratio of fluorescence intensity on the ocular surface (precorneal tear film) and time in the case of using the test solution 1 and the test solution 6.

## Claims

1. An eyedrop wherein the pH thereof is in the range of 4 to 8, comprising 0.001 to 10% (w/v) of hydroxyethylcellulose and 0.001 to 10% (w/v) of dextran, wherein the eyedrop is thickened upon contact with mucin in a precorneal tear film at instillation.

2. The eyedrop according to claim 1, comprising 0.01 to 5% (w/v) of the hydroxyethylcellulose and 0.005 to 3% (w/v) of the dextran.

## Patentansprüche

1. Augentropfen, worin der pH-Wert davon im Bereich von 4 bis 8 liegt, umfassend 0,001 bis 10% (w/v) Hydroxyethylcellulose und 0,001 bis 10% (w/v) Dextran, wobei die Augentropfen nach Kontakt mit Mucin in einem präkornealen Tränenfilm bei Einträufelung verdickt sind.

2. Augentropfen nach Anspruch 1, umfassend 0,01 bis 5% (w/v) Hydroxyethylcellulose und 0,005 bis 3% (w/v) Dextran.

## Revendications

1. Collyre dont le pH se situe dans la plage de 4 à 8, comprenant 0,001 à 10 % (p/v) d'hydroxyéthylcellulose et 0,001 à 10 % (p/v) de dextrane, le collyre s'épaississant lors du contact avec la mucine en un film lacrymal précornéen à l'instillation.

2. Collyre selon la revendication 1, comprenant 0,01 à 5 % (p/v) de l'hydroxyéthylcellulose et 0,005 à 3 % (p/v) du dextrane.

# Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002097129 A

- US 6572849 B

**Non-patent literature cited in the description**

- *Pharmaceutical Research,* 1990, vol. 7 (5), 491-495